# EUROPÄISCHE PATENTSCHRIFT

(11) **EP 2 173 267 B1**
(45) Veröffentlichungstag und Bekanntmachung des Hinweises auf die Patenterteilung: **09.11.2011**
(21) Anmeldenummer: 08785142.4
(22) Anmeldetag: 28.07.2008
(51) Int. Cl.: A61B 17/72

(54) **IMPLANTATVORRICHTUNG ZUR GEWEBE- UND/ODER KNOCHENDISTRAKTION**
IMPLANT DEVICE FOR TISSUE AND/OR BONE DISTRACTION
DISPOSITIF D'IMPLANT POUR DISTRACTION TISSULAIRE ET/OU OSSEUSE

(30) Priorität: 31.07.2007 DE 102007036359
(43) Veröffentlichungstag der Anmeldung: 14.04.2010
(73) Patentinhaber: Storz-Irion, Regina, 78576 Emmingen-Liptingen (DE)
(72) Erfinder: GÜMPER, Paul, 78351 Bodman-Ludwigshafen (DE); STRITTMATTER, Joachim, 78646 Konstanz (DE); BOES, Tobias, Mirco, 75245 Neulingen-Bauschlott (DE); STORZ-IRION, Regina, 78576 Emmingen-Liptingen (DE)
(74) Vertreter: Behrmann, Niels
(86) Internationale Anmeldenummer: PCT/EP2008/006191
(87) Internationale Veröffentlichungsnummer: WO 2009/015847

(56) Entgegenhaltungen:
- WO-A-03/043512
- DE-A1- 19 829 523
- FR-A- 2 726 460
- US-A- 5 575 790
- US-A- 5 961 553

## Beschreibung

Die vorliegende Erfindung betrifft eine Implantatvorrichtung zur Gewebe- und/oder Knochendistraktion.

Eine solche Vorrichtung ist beispielsweise aus der deutschen Patentanmeldung 198 10 639 A1 der Anmelderin bekannt. Eine derartige Vorrichtung, welche langgestreckte Antriebsmittel, Stellmittel, Heizmittel, eine elektrische Energieversorgung mittels drahtloses Energieübertragungs mittel und eine Schaltvorrichtung aufweist, nutzt die besonderen Eigenschaften einer Formgedächtnislegierung (im Weiteren abgekürzt FGL), bei Temperatureinwirkung eine Längenveränderung durchzuführen. Im bekannten Stand der Technik wird diese Längenveränderung dann verwendet, um gezielt schrittweise eine Streckbewegung der gattungsgemäßen Antriebsmittel zu bewirken, dergestalt, dass zwei typischerweise hülsenartige und mit geeigneten Verankerungsabschnitten am zu distrahierenden Knochen oder Gewebe befestigte Elemente gesteuert auseinanderbewegt werden, so dass die beabsichtigte Streckbewegung entsteht.

Während eine derartige Vorrichtung besonders vorteilhaft einen vorbestimmten, schrittweisen Hub der Streckbewegung anbietet, insoweit therapeutisch wirkungsvoll ist, erweist sich diese gattungsgemäße Vorrichtung im praktischen Einsatz noch als verbesserungsbedürftig, vor allem im Hinblick auf eine einfache, betriebssichere Eintragung von Antriebsenergie in die Antriebsmittel, ferner im Hinblick auf eine klare, kausal eindeutige Rückkopplung, ob tatsächlich die Implantatvorrichtung (welche typischerweise als Marknagel in einen Knochen eingeführt werden kann) auch tatsächlich eine Verlängerung durch Streckbewegung erfolgreich durchgeführt hat. Die (typischerweise drahtgebundene) Energieversorgung der Antriebsmittel durch eine extrakorporale Stromversorgungseinheit, welche typischerweise durch Aktivieren bzw. Deaktivieren der Antriebsmittel den Streckbetrieb auslöst, ist zudem fehleranfällig und führt, insbesondere bei unsachgemäßer Bedienung (etwa zu häufiges Aktivieren der Stromversorgung innerhalb eines kurzen Zeitraumes) zu beträchtlichen Komplikationen; da etwa im Fall einer Knochendistraktion nur eine maximale Streckbewegung innerhalb eines vorbestimmten Zeitintervalls (z.B. eines Tages) sinnvoll ist, bedingt durch die Fähigkeit des Körpers zur Callusbildung, besteht zudem das Bedürfnis, einen maximalen Streckhub der Antriebsmittel innerhalb dieses Zeitabstandes vorzubestimmen und darüber hinausgehende Aktivierungen zu verhindern.

Aufgabe der vorliegenden Erfindung ist es daher, eine Implantatvorrichtung nach dem Oberbegriff des Hauptanspruchs zu schaffen, welche im Hinblick auf Einfachheit der elektrischen Energieeintragung, Bedienbarkeit und Steuerbarkeit (insbesondere auch Verhinderung von Fehlbedienungen) verbessert ist, ferner die Grundlage dafür schafft, dass mit lediglich sehr geringem Aufwand und ohne zusätzliche operative Eingriffe extrakorporal eine genaue Überwachung und Kontrolle des (typischerweise eine Mehrzahl von gestuften Streckbewegungen erfordernden) Distraktionsprozesses ermöglicht wird.

Die Aufgabe wird durch die Vorrichtung mit den Merkmalen des Hauptanspruchs gelöst.

In erfindungsgemäß vorteilhafter Weise erfolgt zunächst die elektrische Energieversorgung der die Stellmittel erwärmenden Heizmittel extrakorporal mittels drahtloser Energieübertragungsmittel, bevorzugt auf der Basis elektromagnetischer Wellen (wobei hierfür typischerweise der Implantatvorrichtung eine geeignete, ebenfalls implantierte Spulenvorrichtung zugeordnet ist, welche extrakorporal und drahtlos durch einen geeigneten elektromagnetisch angekoppelten Sendepartner versorgt werden kann). Für die drahtlose HF-Übertragung durch Gewebe ist ein Frequenzbereich von 15 KHz bis 75 KHz aus derzeitiger Sicht sehr gut geeignet.

Ferner erfindungsgemäß ist den Antriebsmitteln eine Schalt- und/oder Sensorvorrichtung zugeordnet, welche eine erfolgte (durch Temperaturveränderung induzierte) Längenveränderung der Stellmittel detektiert bzw. als Reaktion auf die Längenveränderung einen Schaltvorgang auslöst, ergänzend oder alternativ zusätzlich eine tatsächlich erfolgte Streckbewegung, nämlich eine Abstandsänderung zwischen den Verankerungsabschnitten, detektiert (bzw. als Reaktion darauf ebenfalls einen Schaltvorgang auslöst).

Dabei ist es sowohl von der Erfindung umfasst, die Schalt- und/oder Sensorvorrichtung als Einheit mit mindestens einem Schalter durchzuführen, welcher -- geeignet angesteuertals Reaktion auf die Längenveränderung der Stellmittel oder die Streckbewegung einen Schaltvorgang betreffend die Aktivierung oder Deaktivierung der Energieversorgung auslöst, alternativ oder ergänzend die Schalt- und/oder Sensorvorrichtung durch eine Sensorik realisiert ist, welche als Reaktion auf die detektierte Bewegung von Stellmitteln oder Antriebsmitteln entsprechende Signale auslöst, bevorzugt ebenfalls vorbeschriebene Aktivierungs- bzw. Deaktivierungsvorgänge bewirkt.

In erfindungsgemäß vorteilhafter Weise ist es damit möglich, etwa als Reaktion auf einen die Längenveränderung hervorrufenden Wärmeeintrag in die Stellmittel zunächst die weitere Energiezufuhr (durch einen Schaltvorgang) zu deaktivieren, so dass ein weiterer Energieeintrag verhindert wird. Gleichzeitig kann (in ansonsten aus dem Stand der Technik, etwa der gattungsbildenden DE198 10 639 A1 bekannter Weise) das Stellmittel mittels eines Kraftspeichers die Streckbewegung induzieren (bei typischerweise nach wie vor deaktivierter Energiezufuhr zu den Stellmitteln). Erst nach erfolgter Streckbewegung mit dem vorbestimmten (typischerweise durch eine Rastung bzw. Verzahnung bestimmten) Streckhub wird durch erneuten Schalt- bzw. Sensorikvorgang die Energiezufuhr zu den Stellmitteln wieder aktiviert. Im Rahmen der Erfindung finden diese Schaltvorgänge innerhalb des Implantats statt, so dass zur Durchführung des Streckvorgangs eine selbststeuernde Einheit vorliegt. Pro Streckbewegung wird eine elektrische Energie von ca. 240 J bei durchschnittlicher Leistungsaufnahme von ca. 4 Watt benötigt und eingekoppelt; der Streckvorgang dauert typischerweise 1 Min.

Zusätzlich und vorteilhaft bieten die (günstig extern durch entsprechende Energieaufnahme oder Nicht-Energieaufnahme z.B. über das unterschiedliche Anpassungsverhalten der Hochfrequenzübertragungseinheit einfach detektierbaren) Schaltzustände die Möglichkeit, den Erfolg der Distraktionsmaßnahmen wirkungsvoll zu überprüfen: so würde gemäß der Erfindung das extrakorporal mess bzw. detektierbare Deaktivieren der Energiezufuhr zu den Heizmitteln einen Hinweis darauf geben, dass ordnungsgemäß die Längenveränderung der Stellmittel stattgefunden hat; das (typischerweise innerhalb eines vorbestimmten und überwachbaren) Zeitfensters zu detektierende (Wieder-) Aktivieren der Energieversorgung würde dann extern zuverlässig anzeigen, dass auch tatsächlich die Distraktion durch vollständiges Beenden der Streckbewegung stattgefunden hat.

Werden dann noch diesen (bevorzugt extrakorporal einzusetzenden) Detektormitteln weitere Einheiten zur Aufzeichnung bzw. Protokollierung und Überwachung (Monitoring) der ordnungsgemäßen Funktion zugeordnet, lässt sich nicht nur mit einfachsten Mitteln eine zuverlässige Erkennung von Fehlfunktionen vornehmen (und entsprechend ein Alarmsignal ausgeben), auch ist, wichtig etwa für Dokumentationszwecke, eine vollständige Überwachung und Protokollierung des gesamten, typischerweise mehrtägigen Distraktionsprozesses möglich, ohne dass eine geschulte Bedienperson stets präsent sein muss und den Distraktionserfolg überwacht.

Weiterbildungsgemäß weisen die Antriebsmittel eine Rastvorrichtung auf, welche weiter bevorzugt eine bevorzugt regelmäßige Zahnung zum Vorbestimmen einzelner, einem Zahnabstand entsprechender Streckbewegungsschritte bestimmen. Die mechanische Realisierung einer solchen Rastvorrichtung ergibt sich aus der DE198 10 639 A1.

Während zudem eine bevorzugte Realisierungsform der Erfindung vorsieht, dass eine Realisierung der Schalt- und/oder Sensorvorrichtung als mechanischer Schalter mittels Kontaktfeder erfolgt (welche entweder bistabil oder gegen die Kraft einer Rückstellfeder wirkend ausgeführt sein kann), liegt es im Rahmen anderer Weiterbildungen der Erfindung, einen solchen Schalter auch elektronisch zu realisieren, etwa mittels geeigneter Halbleiter-Schaltelemente, welche dann von der eine Sensorik aufweisenden Schalt- und/oder Sensorvorrichtung geeignet angesteuert bzw. betätigt werden.

Weiterbildungsgemäß sieht die Realisierung mittels mechanisch wirksamen Schalters zudem vor, dass an den Stellmitteln und/oder Gehäuseschalen der Antriebsmittel angreifende Schieber bzw. Steuerbleche für eine Betätigung des mindestens einen Schalters sorgen.

Während es zudem bevorzugt ist, sämtliche Schaltvorgänge im Rahmen der vorliegenden Erfindung mittels eines einzelnen Schalters durchzuführen (welcher weiter bevorzugt entweder endseitig in den Antriebsmitteln vorgesehen sein kann, alternativ in einem mittleren Bereich), ist es auch von der Erfindung umfasst, jeder isolierten Schalt- und/oder Detektionsaufgabe eine spezifische Schaltereinheit zuzuordnen, etwa das Schalten als Reaktion auf die Längenänderung der Stellmittel von einem ersten Schalter durchzuführen, während die detektierte Streckbewegung der Antriebsmittel zu einem Schalten einer zweiten Schaltereinheit führt.

Bevorzugt im Rahmen der Erfindung ist es zudem, zur Realisierung der hochfrequenzmäßigen Energieeinkoppelung in die Implantatvorrichtung eine der RFID (radio frequency identification) ähnlichen Technologie anzuwenden, etwa mittels typischer Antennen- und Leiterstrukturen, welche im Hinblick auf effiziente Energieübertragung durch die Haut hindurch in das Implantat optimiert sind.

Weiterbildungsgemäß vorteilhaft ist die erfindungsgemäße Implantatvorrichtung Teil eines Überwachungs- bzw. Monitoringsystems, dergestalt, dass bevorzugt extrakorporale Detektormittel bzw. eine diesen zugeordnete Hinweis- und/oder Alarmeinheit eine kontinuierliche, langfristige Überwachung eines Distraktionsvorgangs ermöglichen, ohne dass medizinisches Fachpersonal anwesend sein muss oder gar invasive Techniken notwendig sind, um einen ordnungsgemäßen Distraktionsfortschritt festzustellen.

Im Ergebnis entsteht durch die vorliegende Erfindung in überraschend einfacher und eleganter Weise die Möglichkeit, vorteilhafte Eigenschaften von Formgedächtnislegierungen und damit realisierte mechanische Antriebsmittel zur Distraktion mit selbst-steuernder Aktivierungs- und Deaktivierungsfunktionalität für die beabsichtigte schrittweise Streckung zu kombinieren, wobei zusätzlich und erfindungsgemäß vorteilhaft die Schalt- (Sensor-)vorgänge als einfache, drahtlose Rückmeldung zur extrakorporalen Überwachung bzw. Erfolgsmessung vorliegen.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Fig. 1:: eine perspektivische Explosionsdarstellung wesentlicher Funktionskomponenten der Implantatvorrichtung gemäß einer ersten, bevorzugten Ausführungsform der vorliegenden Erfindung;
- Fig. 2:: ein schematisches Schaltbild zum Verdeutlichen eines Systems bestehend aus der Implantatvorrichtung gemäß Figur 1, einer damit verbundenen, implantierten Empfangsantenne sowie, extrakorporal gegenüberstehend, eine Sendeantenne zur Energieversorgung samt Steuer- und Detektorelektronik;
- Fig. 3:: eine seitliche Schnittansicht des Bereichs der Formgedächtnislegierung gemäß Figur 1 mit ansitzender Zugstange;
- Fig. 4:: eine Schnittansicht des Feder- und Schieberbereichs der Ausführungsform gemäß Figur 1 mit mittig durchgeführter Zugstange und Illustration der Rastung zur schrittweisen Vorschub- bzw. Streckhubbestimmung;
- Fig. 5:: eine Schnittansicht durch den schalterseitigen Endbereich des Ausführungsbeispiels der Figur 1 samt einem Verankerungsabschnitt zur Befestigung am/im Knochen;
- Fig. 6, Fig. 7:: Schnittansichten durch den endseitigen Schalter der Ausführungsform gemäß Figur 1 im eingeschalteten (Figur 6) bzw. ausgeschalteten Zustand (Figur 7) und
- Fig. 8, Fig. 9:: Ansichten entsprechend Figur 6 bzw. Figur 7, jedoch in um 90° axial gedrehter Schnittansicht;
- Fig. 10:: Blockschaltbild einer Variante eines Gesamtsystems mit den wesentlichen Systemkomponenten; die Version gemäß Anspruch 13 ist gepunktet dargestellt.

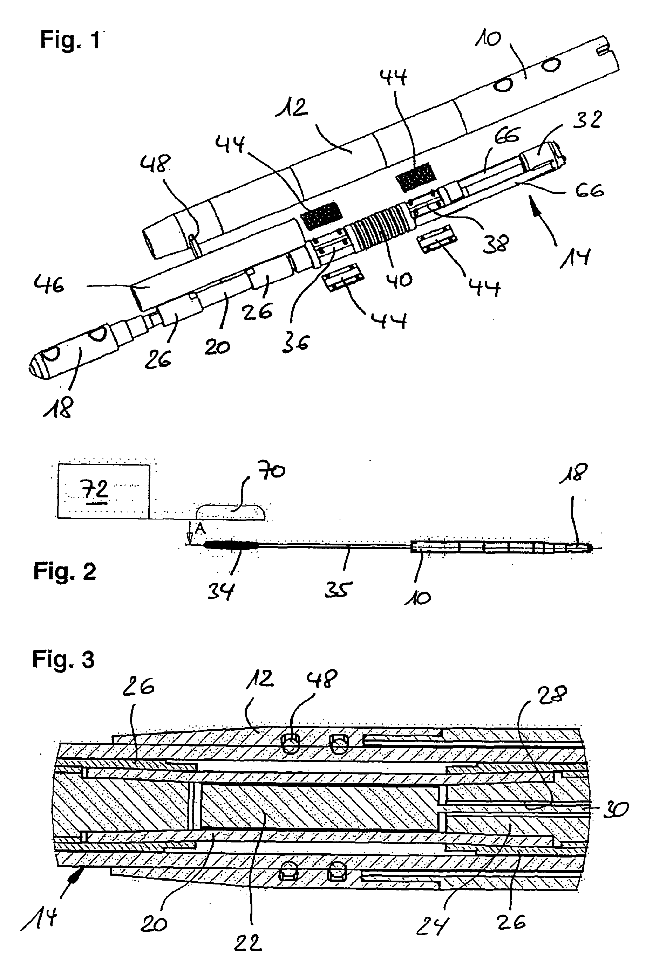

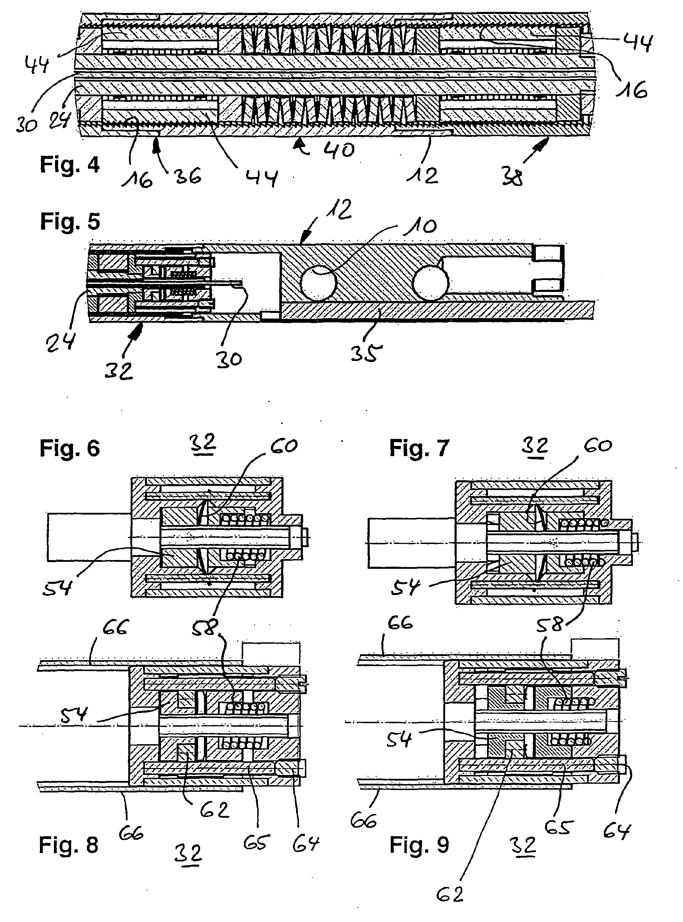

Die Fig. 1 verdeutlicht die erfindungsgemäße Implantatvorrichtung zur Knochendistraktion gemäß einer ersten, bevorzugten Ausführungsform. Wesentliche Funktionselemente, insoweit realisierend die Antriebsmittel der Erfindung, sind eine gegeneinander schrittweise verschiebbare Anordnung aus einem endseitig einen Verankerungsabschnitt 10 aufweisenden Außenrohr 12, welches mit einem darin geführten und relativ zu diesem stufenweise verschiebbaren Innenaggregat 14 so zusammenwirkt, dass -- entlang einer durch eine Verzahnung 16 (Figur 4) bestimmten Rastung -- eine schrittweise Streckung der Aggregate 12 und 14 (durch nachfolgend zu beschreibendes Auseinanderziehen) bewirkt wird, mit dem Ergebnis, dass ein etwa am gegenüberliegenden Verankerungsabschnitt 18 des Innenaggregats 14 befestigtes Knochenfragment relativ zum Abschnitt 10 verschoben und damit gestreckt werden kann. Die wesentliche mechanische Streckbewegung wird dabei induziert von einem Rohr 20 aus einem Formgedächtnis-Legierungsmaterial (hier: NiTi), welches von einem innenliegenden Heizelement 22 (Fig. 3) elektrisch erwärmbar und so ausgebildet ist, dass es bei Erreichen einer Umwandlungstemperatur für das Formgedächtnis-Legierungsmaterial eine Kontraktion durchführt. Diese Bewegung des Rohres 20 wird übertragen auf eine Zugstange 24 (vergleiche Fig. 3 rechts), welche mittels einer Überwurfmutter 26 mit dem hülsenförmigen Formgedächtnis-Legierungselement 20 (als erfindungsgemäßes Stellmittel) verbunden ist und axial mittig einen zentralen Durchbruch 28 zum Führen einer schematisch gezeigten elektrischen Zuleitung 30 für das Heizelement 22 aufweist.

Die Zugstange 24 wirkt im gezeigten Ausführungsbeispiel zusammen mit einer endseitig im Aggregat 14 vorgesehenen Schaltereinheit 32, welche durch die Zugstange 24 in einen Aus- bzw. Einschaltzustand versetzt wird und mit diesen Schaltzuständen eine extern des Außenrohrs 12 und des Innenaggregats 14 im menschlichen Körper implantierte Empfangs-Spuleneinheit 34 für elektrische Energie mit den elektrischen Zuleitungen 30 und 35 für das Heizelement 22 gesteuert verbindet.

Zwischen den in Figur 3 gezeigten Stellmitteln mit Formgedächtnis-Legierungsmaterial und der endseitigen Schaltereinheit 32 ist, gemäß Detailansicht der Fig. 4, eine Anordnung bestehend aus einer ersten Schiebereinheit 36, einer zweiten Schiebereinheit 38 und einer dazwischen liegenden Druckfedereinheit 40 so vorgesehen, dass -- analog der Offenbarung der DE198 10 639 A1 -- eine Bewegung des Rohres 20 zunächst eine Spannung der Federeinheit 40 bewirkt, eine darauf folgende umgekehrte Bewegung des Rohres 20 dann durch Wirkung der Federeinheit 40 und gegen die Sperrwirkung von in die Innenverzahnung 16 des Außenrohres 12 eingreifender (kämmender) Sperrelemente 44, welche (Fig. 4) federnd mit Vorspannung nach außen gelagert sind, eine vorbestimmte Relativbewegung zwischen dem Außenrohr 12 und dem Innenaggregat 14 bewirkt. Im Gegensatz zu dem aus DE 198 10 639 A1 bekannten Stand der Technik, sind das Formgedächtnis-Legierungselement 20 und die Federeinheit 40 räumlich getrennt, während im herangezogenen Stand der Technik diese räumlich einander umgebend und zwischen den Schiebereinheiten dargestellt sind.

Die Fig. 4 verdeutlicht zusätzlich, wie -- zentrisch entlang der Längsachse hindurchgeführt -- die Zugstange 24 sich durch die Anordnung aus erster und zweiter Schiebereinheit 36 und 38 und dazwischenliegender Federeinheit 40 erstreckt, ferner zentrisch durch die Zugstange 24 hindurch die Zuleitung 30 geführt ist.

Ergänzend zeigt die Fig. 3 eine die Anordnung aus Heizelement 22, Formgedächtnislegierungs-Stellmittel 20 (Rohr) sowie daran ansitzendem Ende der Zugstange 24 samt Überwurfmuttern 26 und umgebender Hülse 46, welche mittels einer Ringdichtungsanordnung 48 gegen das umgebende Ende des Außenrohres 12 abgedichtet ist.

Die Fig. 5 verdeutlicht in der Längsschnittansicht den rechtsseitigen Endbereich der Anordnung gemäß Fig. 1, nämlich mit in Schnittansicht gezeigter Schaltereinheit 32, darin eingreifender Zugstange 24 sowie den übergreifenden Endbereich des Außenrohres 12 samt Verankerungsabschnitt 10. Zusätzlich gezeigt ist die im Verankerungsabschnitt 10 integrierte Zuleitung 35, die von der externen (implantierten) Empfangsspuleneinheit (HF-Antenne) 34 herangeführt wird , und es ist, schematisch gezeigt, ein Ende der Zuleitung 30 als aus der Schaltereinheit 32 herausragend gezeigt, welche in nachfolgend zu beschreibender Weise mit der Zuleitung 35 über die Schaltereinheit 32 verbunden ist.

In der geschnittenen Detailansicht der Fig. 6 und 7 bzw. 8 und 9 (um 90° axial verdrehte Schnittansichten der Fig. 6 und 7) wird deutlich, dass, in der Art eines verschiebbaren Ankers, der Schalterabschnitt beweglich in einem Schaltergehäuse gegen die Kraft einer Rückstellfeder 58 geführt ist und eine Knickfeder 60 als Kontaktfeder zwischen einer Ausschaltstellung in Fig. 7 und Fig. 9 sowie einer Einschaltstellung in Fig. 6 und Fig. 8 bewegt wird; kontaktbildend sind dabei insbesondere die in der Einheit 54 bewegten Kontaktabschnitte 62, an welche die Anschlussdrähte der Zuleitungen 30 bzw. 35 über aus der Schaltereinheit 32 herausgeführte externe Anschlüsse 64 und Kontaktstifte 65 angeschlossen sind. Konkret ist die Schalteranordnung der Fig. 6 bis 9 so realisiert, dass als Reaktion auf eine (in der Zeichnungsanordnung nach links gerichtete) Zugkraft der Einheit 24 ein Ausschaltvorgang bewirkt wird (Fig. 7, Fig. 9), während nach Entfall der Zugkraft durch Wirkung der Rückstellfeder 58 der Einschaltzustand (Fig. 6, Fig. 8) wieder hergestellt wird. Maßgeblich hierfür ist die Wirkung des in Fig. 6 bzw. 8 gezeigten beidseitig eingreifenden Paares von Leitblechen 66 (siehe auch Fig. 1), welche zum Eingreifen in und schaltenden Betätigen der Knickfeder 60 einen geeigneten Durchbruch ausbilden.

Der Betrieb der in den Zeichnungen 1 bis 9 verdeutlichten Vorrichtung gemäß einer ersten, primär mechanischen Ausführungsform mit einer einzelnen Schaltereinheit ist wie folgt:

Mittels einer externen Sendespule 70, betrieben durch eine i.w. schematisch gezeigte Ansteuer- und Energieversorgungseinheit 72, wird von extrakorporaler Seite her in Richtung des Pfeils A in die implantierte Empfangsspuleneinheit 34 elektrische Energie zur Zuführung in das Heizelement 22 eingetragen; es wird angenommen, dass in diesem Betriebszustand die Schaltereinheit 32 eingeschaltet ist (Fig. 6, Fig. 8) und entsprechend die Zuleitung 35 von der Empfangsspule 34 zur Zuleitung 30 für die Einleitung der elektrischen Heizenergie durchschleift.

Als Reaktion auf die Erwärmung des Heizelementes 22 führt das Hülsenelement 20 aus NiTi-Formgedächtnismaterial eine Kontraktionsbewegung durch, mit dem Ergebnis, dass das Zugstangenelement 24 eine Zugbewegung erfährt (in der Bilddarstellung der Figur 3 nach links). Diese Zugbewegung überträgt sich zunächst auf die zweite Schiebereinheit 38 (Figur 4 rechts), welche sich um eine Raststufe entsprechend der Verzahnung 16 nach links bewegt und die Federeinheit 40 vorspannt (die Verzahnung 16 wirkt insoweit als Widerlager). Gleichzeitig bewirkt die Zugstange 24 an der endseitigen Schaltereinheit 32 ein Versetzen der Schaltereinheit über die Leitbleche 66 in den ausgeschalteten Zustand (Figur 7, Figur 9); durch Relativbewegung gegen das Steuerblechpaar 66 klappt die Knickfeder 60 in die in Figur 7 bzw. Figur 9 gezeigte Ausschalt-Stellung um, und der elektrische Kontakt zwischen der Empfangsspule 34 und dem Heizelement 22 ist unterbrochen.

Dieses Ausschalten der Energiezufuhr wird in der Ansteuer- und Energieversorgungseinheit 72 detektiert und gespeichert.

Ein nachfolgendes Erkalten des Formgedächtnis-Legierungsmaterials unter die Umwandlungstemperatur der NiTi-Legierung des Rohres 20 führt dann wieder zu einer Abnahme der Zugspannung auf das Element 24, mit der Folge, dass durch Vorspannung der Federeinheit 40 die erste, vordere Schiebereinheit 36 das Außenrohr um eine Rasteinheit in Linksrichtung bewegt, mithin die gewünschte Streckbewegung vornimmt.

Dies führt dann dazu, dass die Schaltereinheit 32 wiederum in den in Fig. 6 und 8 gezeigten Einschaltzustand mit Hilfe der Rückstellfeder 58 versetzt wird, also wiederum in die Spule 34 eingetragene Energie zum Heizelement 22 durchgeleitet wird und damit Bereitschaft für den nächsten Distraktionsschritt besteht.

Diese im Ablauf beschriebene Wirkungskette mittels des Aus- und Einschaltvorgangs ermöglicht vorteilhaft nicht nur die Bewegungssteuerung der Vorrichtung zu den vorgesehenen Distraktionszwecken, auch verhindert das zwischenzeitliche Deaktivieren der Energiezuführung zum Heizelement, dassetwa bei noch nicht ordnungsgemäß erfolgtem mechanischem Vorschub der Antriebsmittel -- eine Fehlbedienung vorgenommen werden kann, und schließlich eignen sich die Ein- und Ausschaltvorgänge auch günstig zur externen Detektion der Betriebszustände bzw. des ordnungsgemäßen Verfahrensablaufs der erfindungsgemäßen Vorrichtung: So ist es nämlich zunächst möglich, überhaupt festzustellen, ob -- durch die Wirkung der Formgedächtnis-Legierungseinheit 20 (Rohr)die beabsichtigte Vorspannung der Federeinheit 40 stattgefunden hat. Extern wäre nämlich in diesem Fall das Ausschalten der Energiezufuhr zum Heizelement 22 durch Wirkung der Schaltereinheit 32 zu detektieren; ein Energieeintrag in die Einheit ist dann nicht möglich. Sollte dagegen, etwa im Fall eines Fehlers oder einer Betriebsstörung, innerhalb einer typischen, vorbestimmten Zeit nach dem externen Aktivieren der Energiezufuhr, dieser Ausschaltschritt nicht zu detektieren sein, würde bereits extern die Energiezufuhr abzuschalten und eine entsprechende Fehlermeldung auszugeben sein.

Entsprechend ist durch Überwachung von extern das ordnungsgemäße Erfolgen des Streckvorgangs (d.h. der Relativbewegung zwischen den Einheiten 12 und 14 durch Wirkung der Federeinheit 40 bzw. der ersten Schiebereinheit 36, wie oben beschrieben), möglich: Wenn nämlich nicht innerhalb wiederum einer extern geeignet vorbestimmten Zeit als Reaktion auf das detektierte Ausschalten ein Wieder-Einschalten der Schaltvorrichtung (Detektieren durch erneute Stromaufnahme der Heizvorrichtung 22) erfolgt, ist von einem diesbezüglichen Fehler der Streckung auszugehen, so dass wiederum ein entsprechendes Alarmsignal ausgelöst werden kann, insbesondere dafür gesorgt werden kann, dass nicht etwa unbeabsichtigt weitere Streckvorgänge induziert werden.

In erfindungsgemäßer Weiterbildung ist der Ansteuerungs- und Energieversorgungs-Einheit 72 nachgeschaltet (oder in diese unmittelbar integriert) eine entsprechende Alarm-, Aufzeichnungs- und/oder Protokolliereinheit, mit welcher nicht nur, in der vorbeschriebenen Weise, entsprechende unbeabsichtigte Abweichungen, insbesondere durch Protokollierung nicht ordnungsgemäßer Zeitabläufe der Ein- und Ausschaltzustände, vorgenommen werden können, auch lässt sich durch geeignete und ansonsten bekannte Datenverarbeitungsmittel eine langfristige Protokollierung aller Schritte und Schaltzustände vornehmen, so dass damit letztendlich auch eine unmittelbare Überwachung des (mehrstufigen) Distraktionsprozesses möglich ist.

Die vorliegende Erfindung ist nicht auf die beschriebene, primär mechanische Wirkungskette und Realisierungsform beschränkt; vielmehr ist es auch von der Erfindung umfasst, etwa die Schaltereinheit, in Verbindung mit der mechanischen Abtastung bzw. Verbindung des Kontraktionszustandes des Rohres 20, auf andere Weise sensorisch abzutasten und entsprechende Schaltvorgänge elektronisch auszulösen und durchzuführen, etwa mittels geeigneter Leistungselektronik und angesteuert durch eine entsprechende Controller-Einheit. Dem Antriebsmittel können Messsensoren, insbesondere Temperatursensoren, Widerstandssensoren oder Längenmesssensoren, zugeordnet sein, deren Information über bidirektional ausgeführte Energie- bzw. Signalübertragungsmittel von intrakorporal nach extrakorporal übertragen wird, so dass als Reaktion auf ein Über- bzw. Unterschreiten vordefinierte Schwellwerte die Energieübertragung bzw. -versorgung bereits extrakorporal abgeschaltet wird. Dies bewirkt im Fehlerfall (z.B. Versagen der Schaltvorrichtung bzw. des Schalters) eine zusätzliche, extrakorporale Abschaltmöglichkeit und stellt damit eine weitere Sicherheitsmaßnahme im Betrieb des Gesamtsystems dar.

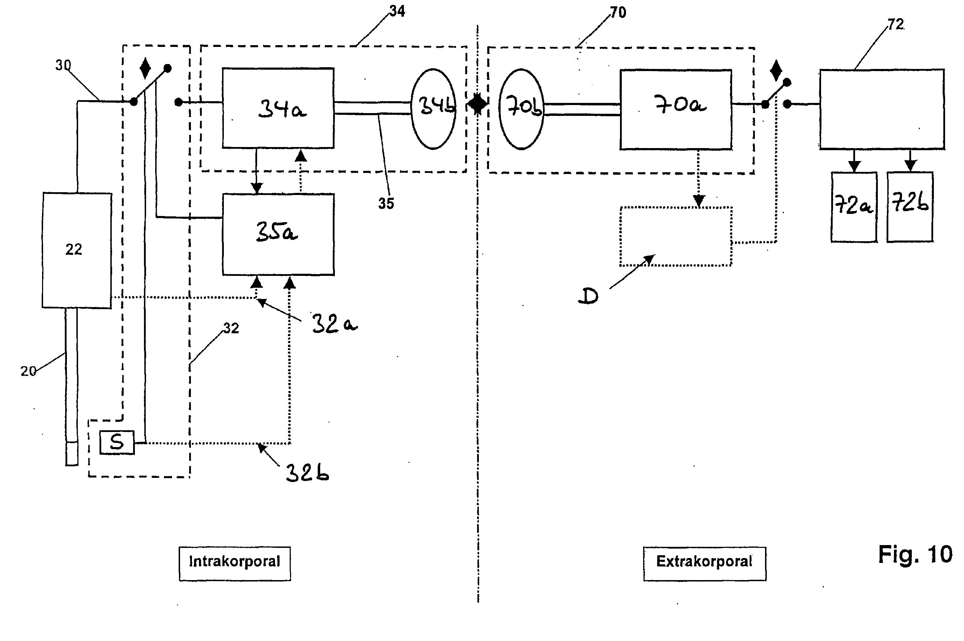

Eine derartige, als mögliche Weiterbildung vorgesehene Systemansicht verdeutlicht die Fig. 10: Geteilt durch die vertikale, doppelt unterbrochene Linie in einen linken intrakorporalen Bereich (also z.B. entsprechend dem Implantationsbereich) und einen rechten extrakorporalen Bereich zeigt die schematische Systemansicht, analog der Fig. 2, das Zusammenwirken der Einheiten 34 und 72, wobei in der in Fig. 10 gezeigten Systemdarstellung eine Steuereinheit 35a die Funktionalität der Einheit 34 in der mit der schematischen Schaltfunktion gezeigten Weise beeinflusst, primär durch Anlegen bzw. Unterbrechen der mittels der Einheiten 20 bzw. 22 realisierten und über die Leitung 30 herangeführten elektrischen Energieversorgung. Die Schalt- und/oder Sensoreinheit 32 ist hier mit einer (fakultativen) Sensorfunktionalität versehen, angedeutet durch die gepunkteten Pfeile 32a (zum Verdeutlichen einer Temperatursensor-Steuerung, bei welcher eine Umklapptemperatur mittels Thermosensor erfasst und an die zentrale Steuereinheit 35a angelegt wird) und 32b (zum Verdeutlichen einer Wege- bzw. Widerstandssensorik S, welche eine Auslenkung des Rohres 20 und/oder dessen schaltzustandsabhängigen elektrischen Widerstandswert der Steuereinheit 35a anlegt). Auf diese Weise lässt sich die in den vorstehenden Ausführungsbeispielen illustrierte reine Schalterfunktionalität der Einheit 32 durch (elektronisch verarbeitbare) Sensorsignale ergänzen bzw. substituieren.

Auf extrakorporaler Seite ist zunächst mittels des gepunktet angedeuteten Detektorpfades D illustriert, wie als Reaktion auf eine detektierte intrakorporale Fehlfunktion unmittelbar die Energieversorgung (bereitgestellt aus Einheit 72) unterbrochen werden kann. Die zugeordneten Einheiten 72a, 72b symbolisieren in der vorbeschriebenen Weise ergänzende Protokoll-, Monitoring-, Alarm- und/oder Hinweisgeberfunktionalitäten, um einen Dauerbetrieb der beschriebenen Vorrichtung zu protokollieren, zu überwachen oder auf andere Weise einer bevorzugt elektronischen Nachbearbeitung zugänglich zu machen, eingeschlossen etwa mittels nicht näher gezeigter drahtloser Telekommunikationsmodule.

## Patentansprüche

1. Implantatvorrichtung zur Gewebe- und/oder Knochendistraktion mit bevorzugt langgestreckten Antriebsmitteln (12, 14), die zum Durchführen einer Streckbewegung zwischen zwei Verankerungsabschnitten (10, 18) für Gewebe und/oder Knochen ansteuerbar sind, wobei die Streckbewegung durch längenveränderliche, ein Formgedächtnislegierungsmaterial aufweisende Stellmittel (20) bewirkt wird, wobei den Stellmitteln (20) zugeordnete Heizmittel (22) und eine elektrische Energieversorgung von den Heizmitteln (22) vorgesehen sind, welche extrakorporal mittels drahtloser Energieübertragungsmittel, auf der Basis hochfrequenter elektromagnetischer Wellen erfolgt, wobei den Antriebsmitteln (12,14) eine Schaltvorrichtung (32) zum Aktivieren und/oder Deaktivieren der Energieversorgung für die Heizmittel so zugeordnet ist, dass als Reaktion auf eine vorbestimmte Längenveränderung der Stellmittel (20) und/oder eine vorbestimmte Streckbewegung der Antriebsmittel (12,14) ein innerhalb der implantatvorrichtung stattfinden der Schaltvorgang der Schaltvorrichtung (32) ausgelöst wird, wobei die Schaltvorrichtung (32) mindestens einen mechanisch von den Stellmitteln (20) oder den Antriebsmitteln (12,14) betätigten Schalter (32) aufweist, der in einen Heizstromkreis zwischen einer Energieempfangseinheit (34) und den Heizmitteln (22) eingeschleift ist und wobei der Schalter (32) durch eine in der Richtung der Streckbewegung bewegbare Einheit, aufweisend eine Zugstange (24) und/oder ein Leitblech (66), betätigbar ausgebildet ist.

2. Vorrichtung nach Anspruch 1, **dadurch gekennzeichnet, dass** die Schaltvorrichtung (32) so ausgebildet ist, dass diese als Reaktion auf die vorbestimmte Längenveränderung der Stellmittel (20) die Energieversorgung für die Heizmittel deaktivieren und/oder als Reaktion auf die vorbestimmte Streckbewegung der Antriebsmittel (12,14) die Energieversorgung aktivieren kann.

3. Vorrichtung nach Anspruch 1 oder 2, **dadurch gekennzeichnet, dass** die Antriebsmittel (12,14) eine bevorzugt regelmäßig gezahnte Rastvorrichtung (16, 36, 38, 44) aufweisen, die eine schrittweise Streckbewegung entsprechend einem jeweiligen Rast- und/oder Zahnabstand der Rastvorrichtung (16, 36, 38, 44) bestimmt.

4. Vorrichtung nach Anspruch 3, **dadurch gekennzeichnet, dass** die den Schaltvorgang der Schaltvorrichtung (32) auslösende vorbestimmte Streckbewegung der Antriebsmittel (12,14) einem Schritt oder einer vorbestimmten Mehrzahl von Schritten entlang der Rastvorrichtung entspricht.

5. Vorrichtung nach Anspruch 1, **dadurch gekennzeichnet, dass** der Schalter (32) eine zwischen zwei Schaltstellungen bewegbare Knickfeder (60) aufweist.

6. Vorrichtung nach einem der Ansprüche 1 bis 5, **dadurch gekennzeichnet, dass** die drahtlosen Energieübertragungsmittel eine Hochfrequenz-Sendeeinheit (72, 70) aufweisen, die in einem implantierten Betriebszustand der Implantatvorrichtung zum Zusammenwirken mit einer Hochfrequenz-Empfangseinheit (34) der Implantatvorrichtung ausgebildet ist und Detektormittel (D) aufweist, die senderseitig eine draht- und/oder berührungslose Feststellung eines Schaltzustands der Schaltvorrichtung (32), insbesondere eine Aktivierung und/oder Deaktivierung der Energieversorgung für die Heizmittel (22), ermöglichen.

7. Vorrichtung nach Anspruch 6, **dadurch gekennzeichnet, dass** den Detektormitteln eine Hinweis- und/oder Alarmeinheit zugeordnet ist, die so ausgebildet ist, dass als Reaktion auf ein Über- und/oder Unterschreiten vorbestimmter, bevorzugt zeitbezogener Schwellwerte zwischen zwei detektierten, aufeinanderfolgender Änderungen des Schaltzustands ein Hinweis- und/oder Alarmsignal erzeugt wird.

8. Vorrichtung nach Anspruch 6 oder 7, **dadurch gekennzeichnet, dass** den Detektormitteln (D) eine Vorrichtung (72) zur elektronischen Erfassung und Aufzeichnung einer Mehrzahl aufeinanderfolgender detektierter Schaltzustände und zur Ausgabe und/oder Speicherung entsprechender elektronisch verarbeitbarer Datensätze zugeordnet ist.

9. Vorrichtung nach einem der Ansprüche 1 bis 8, **dadurch gekennzeichnet, dass** die Antriebsmittel eine langgestreckte, mittels verschiebbarer Hülsen realisierte Gehäuseeinheit, insbesondere bestehend aus Außenrohr und einem Innenaggregat (12, 14), aufweisen, welche zur Aufnahme der Stellmittel (20) und der Heizmittel (22) sowie zum Zusammenwirken mit einer zur empfangsseitigen Energieversorgung als Hochfrequenzantenne ausgebildeten Empfangsspuleneinheit (34) ausgebildet ist.

10. Vorrichtung nach einem der Ansprüche 1 bis 9, **dadurch gekennzeichnet, dass** den Antriebsmitteln (12,14) eine elektronische Steuereinheit zugeordnet ist, welche als Reaktion auf ein von extrakorporal drahtlos eingekoppeltes Steuersignal, insbesondere Hochfrequenz-Steuersignal, ein Aktivieren und/oder Deaktivieren der Energieversorgung für die Heizmittel (22) vornimmt, und welche zum Durchführen eines Funktionstests der Antriebsmittel (12, 14) und/oder zum Aussenden von Status- und/oder Störsignalen ausgebildet ist.

11. Vorrichtung nach einem der Ansprüche 1 bis 10, **dadurch gekennzeichnet, dass** die Schaltvorrichtung zumindest einen Messsensor, insbesondere einen Temperatursensor, aufweist, dessen Information über ein bidirektional ausgeführtes Informationsübertragungsmittel nach extrakorporal übertragen wird und das als Reaktion auf ein Überschreiten vorbestimmter Schwellwerte die Energieversorgung extrakorporal abschaltet.

12. Vorrichtung nach einem der Ansprüche 1 bis 11, **dadurch gekennzeichnet, dass** die Schaltvorrichtung zumindest einen Messsensor, insbesondere einen Widerstandssensor, aufweist, der zur Detektion einer bei einer Phasenumwandlung des Formgedächtnislegierungsmaterials entstehenden Widerstandsänderung ausgebildet ist, wobei bevorzugt Mittel zum Übertragen der Messsensorinformation nach extrakorporal vorgesehen sind, die als Reaktion auf ein Überschreiten eines vorbestimmten Schwellwerts die Energieversorgung extrakorporal abschalten.

## Claims

1. Implant device for tissue and/or bone distraction comprising preferably elongate drive means (12, 14) which can be activated for carrying out an extension movement between two anchoring portions (10, 18) for tissue and/or bone, wherein the extension movement is produced via adjustment means (20) which are variable in length and comprise a shape memory alloy material, heating means (22) associated with the adjustment means (20) and an electrical energy supply of the heating means (22) being provided, which energy supply takes place outside the body via wireless energy transfer means, on the basis of high-frequency electromagnetic waves, a switching device (32) for activating and/or deactivating the energy supply for the heating means being associated with the drive means (12, 14) in such a way that a switching operation, occurring inside the implant device, of the switching device (32) is triggered in response to a predetermined change in the length of the adjustment means (20) and/or a predetermined extension movement of the drive means (12, 14), the switching device (32) comprising at least one switch (32) which is operated mechanically by the adjustment means (20) or the drive means (12, 14) and is looped into a heating current circuit between an energy receiving unit (34) and the heating means (22) and wherein the switch (32) is formed such that it can be operated by a unit which is movable in the direction of the extension movement and comprises a connecting rod (24) and/or a baffle plate (66).

2. Device according to claim 1, **characterised in that** the switching device (32) is formed such that it can deactivate the energy supply for the heating means in response to the predetermined change in length of the adjustment means (20) and/or activate the energy supply in response to the predetermined extension movement of the drive means (12, 14).

3. Device according to either claim 1 or claim 2, **characterised in that** the drive means (12, 14) comprise a preferably uniformly toothed latching device (16, 36, 38, 44) which determines a stepwise extension movement according to a respective latch and/or tooth spacing of the latching device (16, 36, 38, 44).

4. Device according to claim 3, **characterised in that** the predetermined extension movement of the drive means (12, 14), which extension movement triggers the switching operation of the switching device (32), corresponds to a step or a predetermined plurality of steps along the latching device.

5. Device according to claim 1, **characterised in that** the switch (32) comprises a buckling spring (60) which is movable between two switching positions.

6. Device according to any one of claims 1 to 5, **characterised in that** the wireless energy transfer means comprise a high-frequency transmitting unit (72, 70) which is formed for cooperation with a high-frequency receiving unit (34) of the implant device in an implanted operating state of the implant device and comprises detector means (D) which make possible wireless and/or contactless determination on the transmitting side of a switching state of the switching device (32), in particular an activation and/or deactivation of the energy supply for the heating means (22).

7. Device according to claim 6, **characterised in that** an information and/or alarm unit is associated with the detector means, which information and/or alarm unit is formed in such a way that an information and/or alarm signal is generated in response to predetermined, preferably time-related thresholds between two detected successive changes in the switching state being exceeded or undershot.

8. Device according to either claim 6 or claim 7, **characterised in that** a device (72) for electronically detecting and recording a plurality of successive detected switching states and for outputting and/or storing corresponding electronically processible sets of data is associated with the detector means (D).

9. Device according to any one of claims 1 to 8, **characterised in that** the drive means comprise an elongate housing unit, produced by means of displaceable sleeves and in particular consisting of an outer tube, and an inner assembly (12, 14), which is formed for receiving the adjustment means (20) and the heating means (22) and for cooperation with a receiving coil unit (34) formed as a high-frequency antenna for the receiving-side energy supply.

10. Device according to any one of claims 1 to 9, **characterised in that** an electronic control unit is associated with the drive means (12, 14), which control unit activates and/or deactivates the energy supply for the heating means (22) in response to a control signal coupled-in wirelessly from outside the body, in particular a high-frequency control signal, and which is formed for carrying out a function test of the drive means (12, 14) and/or for emitting status and/or trouble signals.

11. Device according to any one of claims 1 to 10, **characterised in that** the switching device comprises at least one measurement sensor, in particular a temperature sensor, the information of which is transferred outside the body via a bidirectional information transfer means and which switches off the energy supply outside the body in response to predetermined thresholds being exceeded.

12. Device according to any one of claims 1 to 11, **characterised in that** the switching device comprises a measurement sensor, in particular a resistance sensor, which is formed for detection of a change in resistance occurring during a phase transition of the shape memory alloy material, means for transferring the measurement sensor information outside the body preferably being provided, which means switch off the energy supply outside the body in response to predetermined thresholds being exceeded.

## Revendications

1. Dispositif d'implant pour distraction tissulaire et/ou osseuse comprenant des moyens d'entraînement (12, 14) de préférence étirés en longueur qui peuvent être pilotés pour effectuer un mouvement d'étirement entre deux sections d'ancrage (10, 18) pour tissu et/ou os, le mouvement d'étirement étant provoqué par des moyens de réglage (20) à longueur modifiable présentant un matériau en alliage à mémoire de forme, des moyens de chauffage (22) attribués aux moyens de réglage (20) et une alimentation en énergie électrique des moyens de chauffage (22) étant prévus, laquelle est effectuée de manière extracorporelle par le biais de moyens de transmission de l'énergie sans fil sur la base d'ondes électromagnétiques à haute fréquence, un dispositif de commutation (32) pour activer et/ou désactiver l'alimentation en énergie pour les moyens de chauffage étant attribué aux moyens d'entraînement (12, 14), de sorte qu'en réaction à une modification de longueur prédéterminée des moyens de réglage (20) et/ou à un mouvement d'étirement prédéterminé des moyens d'entraînement (12, 14), une procédure de commutation du dispositif de commutation (32) ayant lieu à l'intérieur du dispositif d'implant, est déclenchée, le dispositif de commutation (32) présentant au moins un commutateur (32) actionné mécaniquement par les moyens de réglage (20) ou les moyens d'entraînement (12, 14) qui est bouclé dans un circuit de courant de chauffage entre une unité réceptrice d'énergie (34) et les moyens de chauffage (22), et le commutateur (32) étant formé en pouvant être actionné par une unité mobile dans le sens du mouvement d'étirement, présentant une barre de traction (24) et/ou une tôle-guide (66).

2. Dispositif selon la revendication 1, **caractérisé en ce que** le dispositif de commutation (32) est ainsi formé que celui-ci peut, en réaction à la modification de longueur prédéterminée des moyens de réglage (20), désactiver l'alimentation en énergie pour les moyens de chauffage, et/ou en réaction à un mouvement d'étirement prédéterminé des moyens d'entraînement (12, 14), activer l'alimentation en énergie.

3. Dispositif selon la revendication 1 ou 2, **caractérisé en ce que** les moyens d'entraînement (12, 14) présentent un dispositif d'encliquetage (16, 36, 38, 44) de préférence à indentation régulière, qui définit un mouvement d'étirement pas à pas correspondant à un écart d'encliquetage et/ou d'indentation du dispositif d'encliquetage (16, 36, 38, 44).

4. Dispositif selon la revendication 3, **caractérisé en ce que** le mouvement d'étirement prédéterminé des moyens d'entraînement (12, 14) déclenchant la procédure de commutation du dispositif de commutation (32) correspond à un pas ou à une pluralité prédéterminée de pas le long du dispositif d'encliquetage.

5. Dispositif selon la revendication 1, **caractérisé en ce que** le commutateur (32) présente un ressort de flambage (60) mobile entre deux positions de commutation.

6. Dispositif selon l'une des revendications 1 à 5, **caractérisé en ce que** les moyens de transmission de l'énergie sans fil présentent une unité émettrice à haute fréquence (72, 70) qui est formée, dans un état de fonctionnement implanté du dispositif d'implant, pour interagir avec une unité réceptrice à haute fréquence (34) du dispositif d'implant et présente des moyens de détecteur (D) qui permettent côté émetteur une vérification sans contact et/ou sans fil d'un état de commutation du dispositif de commutation (32), en particulier une activation et/ou une désactivation de l'alimentation en énergie pour les moyens de chauffage (22).

7. Dispositif selon la revendication 6, **caractérisé en ce qu'**une unité d'avertissement et/ou d'alarme est attribuée aux moyens de détecteur, laquelle est formée de sorte qu'en réaction au fait que des valeurs seuil prédéterminées, de préférence par rapport au temps, sont dépassées et/ou non atteintes entre deux modifications successives détectées de l'état de commutation, un signal d'avertissement et/ou d'alarme soit émis.

8. Dispositif selon la revendication 6 ou 7, **caractérisé en ce qu'**un dispositif (72) est attribué aux moyens de détecteur (D) pour la détection et l'enregistrement électroniques d'une pluralité d'états de commutation successifs détectés et pour la fourniture et/ou la sauvegarde de jeux de données correspondants pouvant être traités électroniquement.

9. Dispositif selon l'une des revendications 1 à 8, **caractérisé en ce que** les moyens d'entraînement présentent une unité de logement étirée en longueur, réalisée au moyen de douilles déplaçables, se composant en particulier d'un tube extérieur et d'un groupe interne (12, 14), laquelle est formée pour recevoir les moyens de réglage (20) et les moyens de chauffage (22) ainsi que pour interagir avec une unité de bobine réceptrice (34) formée comme une antenne haute fréquence pour l'alimentation en énergie côté réception.

10. Dispositif selon l'une des revendications 1 à 9, **caractérisé en ce qu'**une unité de commande électronique est attribuée aux moyens d'entraînement (12, 14), laquelle, en réaction à un signal de commande couplé sans fil de manière extracorporelle, en particulier un signal de commande haute fréquence, effectue une activation et/ou une désactivation de l'alimentation en énergie pour les moyens de chauffage (22) et laquelle est formée pour exécuter un test de fonctionnement des moyens d'entraînement (12, 14) et/ou pour envoyer des signaux de statut et/ou parasites.

11. Dispositif selon l'une des revendications 1 à 10, **caractérisé en ce que** le dispositif de commutation présente au moins un capteur de mesure, en particulier un capteur de température, dont les informations sont transmises de manière extracorporelle par un moyen de transmission des informations conçu bidirectionnel et qui, en réaction à un dépassement de valeurs seuils prédéterminées, coupe l'alimentation en énergie de manière extracorporelle.

12. Dispositif selon l'une des revendications 1 à 11, **caractérisé en ce que** le dispositif de commutation présente au moins un capteur de mesure, en particulier un capteur de résistance, qui est formé pour la détection d'une modification de résistance se produisant lors d'un changement de phase du matériau en alliage à mémoire de forme, dans lequel de préférence, des moyens de transmission des informations de mesure de manière extracorporelle sont prévus qui, en réaction à un dépassement d'une valeur seuil prédéterminée, coupent l'alimentation en énergie de manière extracorporelle.
